## Europäisches Patentamt
## European Patent Office
## Office européen des brevets

(19)

(11) Numéro de publication: **0 267 066**
**B1**

(12)

# FASCICULE DE BREVET EUROPEEN

(45) Date de publication du fascicule du brevet:
**31.01.90**

(51) Int. Cl.⁴: **C07F 3/02**

(21) Numéro de dépôt: **87402155.3**

(22) Date de dépôt: **28.09.87**

(54) **Nouveaux organomagnésiens sous forme solide, leur procédé de préparation et leur utilisation.**

(30) Priorité: **03.10.86 FR 8613809**

(43) Date de publication de la demande:
**11.05.88 Bulletin 88/19**

(45) Mention de la délivrance du brevet:
**31.01.90 Bulletin 90/5**

(84) Etats contractants désignés:
**AT BE CH DE ES FR GB GR IT LI LU NL SE**

(56) Documents cités:
**EP-A- 0 016 673**

(73) Titulaire: **RHONE-POULENC CHIMIE, 25, quai Paul Doumer, F-92408 Courbevoie Cédex(FR)**

(72) Inventeur: **Corriu, Robert, 246, rue de l'Espéron, F-34100 Montpellier(FR)**
Inventeur: **Cerveau, Geneviève, Clos Saint Joseph C 749, avenue du Pic Saint Loup, F-34100 Montpellier(FR)**
Inventeur: **Chuit, Claude, Route de Sommières, F-30250 Junas(FR)**
Inventeur: **Reye, Catherine, 160, rue d'Alco, F-34100 Montpellier(FR)**
Inventeur: **Boudin, Alain, Rue Flemming Le Souc, F-34800 Clermont l'Herault(FR)**

(74) Mandataire: **Le Pennec, Magali et al, RHONE-POULENC INTERSERVICES Service Brevets Chimie 25, Quai Paul Doumer, F-92408 Courbevoie Cédex(FR)**

# EP 0 267 066 B1

**Description**

La présente invention concerne de nouveaux complexes organomagnésiens, leur procédé de préparation et leurs applications.

Il est connu de préparer les organomagnésiens selon une méthode désormais classique. Ces composés très réactifs, parfois même très violents ne sont stables et transportables qu'en solution dans l'éther ou dans des solvants éthérés, ce qui pose de gros problèmes de sécurité au niveau industriel. Or ils sont utiles pour la synthèse de nouveaux produits organiques en particulier pour la formation de nouvelles liaisons carbone-carbone, il est absolument nécessaire de les fabriquer au fur et à mesure de leur utilisation.

La présente invention a pour but a pallier les inconvénients de la préparation extemporanée des organomagnésiens, elle propose des composés organomagnésiens solides, stables et stockables sous une forme directement commercialisable. Elle a pour objet de nouveaux complexes organomagnésiens solides caractérisés en ce qu'il répondent à la formule générale (I) ci-dessous :

$(RMgX)_m / N [-CHR_1-CHR_2-O-(CHR_3-CHR_4-O)_n R_5]_3 (I)$

dans laquelle:

* m est égal ou supérieur à 1 et égal ou inférieur à 3.
* R représente un groupement :
- alkyle, linéaire ou ramifié comportant de 1 à 18 atomes de carbone,
- alcényle ou alcynyle comportant de 2 à 18 atomes de carbone,
- cycloalkyle ou cycloalcényle comportant de 3 à 12 atomes de carbone,
- alcoxyalkyle, dialcoxyalkyle, alkylthioalkyle,
- aryle éventuellement substitué au moins par un groupe halogène, alkyle, alcoxy, trifluorométhyle, trifluorométhoxy, trifluorométhylthio,
- alcoxyaryle, alkylthioaryle.
* X représente un atome de chlore, d'iode ou de brome.
* n est un nombre entier supérieur ou égal à O et inférieur ou égal à 10 environ ($0 \leq \alpha\mu\rho^{\ddot{}}$ n $\leq \alpha\mu\rho^{\ddot{}}$ 10), $R_1$, $R_2$, $R_3$ et $R_4$ identiques ou différents représentent un atome d'hydrogène ou un radical alkyle ou cycloalkyle ayant de 1 à 4 atomes de carbone, et $R_5$ représente un radical alkyle ou cycloalkyle ayant 1 à 12 atomes de carbone, un radical phényl ou un radical de formule $-C_pH_{2p+1}-\varnothing$ ou $-C_pH_{2p+1}-\varnothing-$, p étant compris entre 1 et environ 12.

Les composés préférentiels sont ceux pour lesquels au moins l'un des critères suivants est rempli :
* R représente un groupement phényle, alkyle, alcényle, comportant au moins 3 atomes de carbone éventuellement substitué par un groupe alcoxy, dialcoxy, ou alkylthio.
* $R_1$, $R_2$, $R_3$, $R_4$ identiques ou différents représentent un atome d'hydrogène ou un radical méthyle.
* n est égal à 0, 1, 2 ou 3.
* $R_5$ représente un groupe alkyle contenant 1 à 4 atomes de carbone.

On peut citer parmi l'ensemble des agents complexants préférentiels :
- la tris(oxa-3 butyl)amine de formule :
$N(CH_2-CH_2-O-CH_3)_3$
- la tris(oxa-3 heptyl)amine de formule :
$N(CH_2-CH_2-O-C_4H_9)_3$
- la tris(dioxa-3,6 heptyl)amine de formule :
$N(CH_2-CH_2-O-CH_2-CH_2-O-CH_3)_3$
- la tris(trioxa-3,6,9 décyl)amine de formule :
$N(CH_2-CH_2-O-CH_2-CH_2-O-CH_2-CH_2-O-CH_3)_3$
la tris(dioxa-3,6, octyl)amine de formule :
$N(CH_2-CH_2-O-CH_2-O-C_2H_5)_3$
- la tris(trioxa-3,6,9 undécyl)amine de formule :
$N(CH_2-CH_2-O-CH_2-CH_2-O-CH_2-CH_2-O-C_2H_5)_3$
- la tris(dioxa-3,6, nonyl)amine de formule :
$N(CH_2-CH_2-O-CH_2-CH_2-O-C_3H_7)_3$
- la tris(trioxa-3,6,9 dodécyl)amine de formule :
$N(CH_2-CH_2-O-CH_2-CH_2-O-CH_2-CH_2-O-C_3H_7)_3$
- la tris(dioxa-3,6, décyl)amine de formule :
$N(CH_2-CH_2-O-CH_2-CH_2-O-C_4H_9)_3$
- la tris(,6,9 tridécyl)amine de formule :
$N(CH_2-CH_2-O-CH_2-CH_2-O-CH_2-CH_2-O-C_4H_9)_3$
- la tris(tétraoxa-3,6,9,12 tridécyl)amine de formule :
$N(CH_2-CH_2-O-(CH_2-CH_2-O-)_3-CH_3)_3$
- la tris(dioxa-3,6, méthyl-4 heptyl)amine de formule :
$N(CH_2-CH_2-O-CHCH_3-CH_2-O-CH_3)_3$
- la tris(dioxa-3,6, diméthyl-2,4 heptyl)amine de formule :
$N(CH_2-CHCH_3-O--CHCH_3-CH_2-O-CH_3)_3$

2

On préfère tout particulièrement les composés de formule générale :

RMgX / N(CH₂-CH₂-O-CH₂-CH₂-O-CH₃)₃

dans lesquels R et X ont la même signification que précédemment

Les nouveaux complexes organomagnésiens de la présente invention sont notamment préparés par réaction de l'agent complexant de formule :

N[-CHR₁-CHR₂O-(CHR₃-CHR₄-O) n R₅]₃

avec le composé organomagnésien de formule RMgX en milieu solvant anhydre et sous atmosphère inerte.

Selon un mode préférentiel de préparation des composés objet de l'invention on introduit dans un solvant de préférence éthéré l'agent complexant puis le composé organomagnésien. Le composé organomagnésien complexé précipite alors et est récupéré par tout moyen connu de l'homme de l'art. On sèche ensuite le composé sous vide et sa conservation est effectuée de préférence sous gaz inerte notamment sous azote. La mise en contact du composé organomagnésien et de l'agent complexant est réalisée avantageusement à la température ambiante. Lors de la préparation des composés selon l'invention on préfère généralement travailler avec un excès de dérivés organomagnésiens. Ainsi un rapport molaire dérivé organomagnésien à l'agent complexant d'au moins 2 est souvent préféré.

D'autres caractéristiques et avantages apparaîtront au cours de la description détaillée de l'invention complétée par des exemples non limitatifs de réalisation.

Le composé organomagnésien-agent complexant peut être synthétisé de la façon suivante :

La préparation est notamment effectuée sous atmosphère neutre, par exemple sous azote.

Trois équivalents de RMgX en solution dans l'éther éthylique sont placés dans un tube de Schlenk sous agitation. On ajoute goutte à goutte un équivalent d'agent complexant dilué dans environ son volume d'éther éthylique à la température ambiante. Dès le début de l'addition, il y a formation d'un précipité blanc qui se transforme en une pâte en fin d'addition.

Ce résidu solide pâteux est filtré, lavé avec de l'éther anhydre, et séché sous vide pendant 2 heures. On obtient ainsi une poudre blanche qui est conservée sous azote.

Cette poudre est dosée et contient 1 à 2 mmoles de composé organomagnésien RMgX par gramme. L'éther de lavage et le filtrat sont également dosés et contiennent le reste de composé organomagnésien.

Il est également possible d'utiliser comme matière première un organomagnésien en solution dans le tétrahydrofuranne (THF) mais il sera avantageux alors d'augmenter la dilution de l'agent complexant.

Les poudres obtenues sont stables jusqu'à environ 120°C.

La présente invention se rapporte également à l'application des complexes de formule I comme réactif de Grignard en vue de synthétiser de nouveaux composés organiques par la formation de nouvelles liaisons carbone-carbone. Pour cela, on fait réagir un complexe de formule I avec un composé carbonylé en milieu solvant organique.

A titre d'exemple de composés carbonyles, on mentionnera les cétones, les aldéhydes, les esters, les chlorures et anhydrides d'acide. A titre de solvant utilisé pour cette application, on mentionnera sans restriction l'éther éthylique, le tétrahydrofuranne, l'éther de pétrole, le cyclohexane, le toluène, l'acétonitrile, les alcanes, le diméthylacétamide, l'hexaméthylphosphoramide, le diméthylformamide.

Par rapport aux réactifs organomagnésiens non complexés ou réactifs de Grignard on note un fort ralentissement de la réaction. Ainsi en fonction de la température réactionnelle on peut faire réagir l'organomagnésien sur une fonction et non sur une autre quand on utilise un agent de condensation multifonctionnel (comportant par exemple une fonction aldéhyde et une fonction ester).

Les exemples ci-après servent à illustrer l'utilisation des composés selon l'invention.

**Exemple I** : Réaction avec les cétones de RMgX / tris(dioxa − 3,6 heptyl)amine

TABLEAU I

| R | X | Réactif | Solvant | Conditions opératoires | Rendement | Produit formé |
|---|---|---|---|---|---|---|
| Phényle | Br | Ø−C(=O)−CH₃ | Ether de pétrole | 70°C / 4 heures | 80 % | Ø−C(OH)(CH₃)−Ø (1) |
| idem | Br | idem | Cyclohexane | 60°C / 4 heures | 60 % | idem |
| idem | Br | idem | Toluène | 60°C / 4 heures | 80 % | idem |
| Allyle | Br | idem | Ether de pétrole | 55°C / 4 heures | 100 % | CH₂=CH−CH₂−C(OH)(CH₃)−Ø (1) |
| Isopropyle | Br | idem | idem | 70°C / 4 heures | 60 % | (CH₃)₂CH−C(OH)(CH₃)−Ø (1) |
| n-Butyle | Br | idem | idem | 70°C / 4 heures | 80 % | n-C₄H₉−C(OH)(CH₃)−Ø (1) |
| Ethyle | Br | idem | Ether éthylique | 20°C / 1 heure | | C₂H₅−C(OH)(CH₃)−Ø (1) |
| Ethyle | Br | idem | THF | −35°C / 15 min | | C₂H₅−C(OH)(CH₃)−Ø (1) |

Exemple II : Réaction avec les esters de RMgX /tris (dioxa – 3,6 heptyl)amine

**TABLEAU II**

| R | X | Réactif | Solvant | Conditions opératoires | Rendement | Produit formé |
|---|---|---------|---------|------------------------|-----------|---------------|
| Ethyle | Br | Benzoate d'Ethyle | THF | 20°C / 1 heure | 100 % | Ø-C(OH)(Et)-Et |
| Ø | Br | Chloroformiate d'Ethyle | Ether éthylique | 20°C / 12 heures | 45 % | Ø-C(=O)-OEt |
| n-Heptyle | Br | idem | idem | idem | 45 % | nHept-C(=O)-OEt |
| Ø | Br | idem | TAF | –15°C / 1 heure | 94 % | Ø-C(=O)-OEt |
| n-Butyle | Br | idem | idem | idem | 30 % | C(=O)-OEt |

EP 0 267 066 B1

Exemple III : Réaction avec les chlorures d'acides de RMgX /tris (dioxa-3,6 heptyl) amine

TABLEAU III

| R | X | Réactif | Solvant | Conditions expérimentales | Rendement | Produit formé |
|---|---|---------|---------|---------------------------|-----------|---------------|
| Ethyle | Br | Chlorure de benzoyle | THF | 0°C | 80 % | $\emptyset-\underset{\underset{O}{\|\|}}{C}-\emptyset$ |

EP 0 267 066 B1

**Revendications**

1 °) Complexes organomagnésiens solides, caractérisés en ce qu'ils répondent à la formule générale :
(RMgX) $_m$/ N [-CHR$_1$-CHR$_2$-O-(CHR$_3$-CHR$_4$-O) $_n$ R$_5$]$_3$
dans laquelle :
* m est égal ou supérieur à 1 et égal ou inférieur à 3.
* R représente un groupement :
- alkyle, linéaire ou ramifié comportant de 1 à 18 atomes de carbone,
- alcényle ou alcynyle comportant de 2 à 18 atomes de carbone,
- cycloalkyle ou cycloalcényle comportant de 3 à 12 atomes de carbone,
- alkoxyalkyle, dialcoxyalkyle, alkylthiocarbonyle,
- aryle éventuellement substitué en position au moins par un halogène, un groupement alcoxy, trifluorométhyle, trifluorométhoxy, trifluorométhylthio,
- alcoxyaryle, alkylthioaryle,
* X represente un atome de chlore, d'iode ou de brome.
* n est un nombre entier supérieur ou égal à O et inférieur ou égal à 10 environ (O ≦αμρ̈ n ≦αμρ̈ 10), R$_1$, R$_2$, R$_3$ et R$_4$ identiques ou différents représentent un atome d'hydrogène ou un radical alkyle ou cycloalkyle ayant de 1 à 4 atomes de carbone, et R$_5$ représente un radical alkyle ou cycloalkyle ayant 1 à 12 atomes de carbone, un radical phényle ou un radical de formule -C$_p$H$_{2p}$-∅ ou C$_p$H$_{2p+1}$-∅-, p étant compris entre 1 et environ 12.

2 °) Complexes organomagnésiens solides selon la revendication 1, caractérisé en ce que :
R représente un groupement alkyle, alcényle comportant au moins 3 atomes de carbone éventuellement substitué par un groupe alcoxy, dialcoxy, alkylthio ou phényle.

3 °) Complexes organomagnésiens solides selon la revendication 1, caractérisé en ce que :
R$_1$, R$_2$, R$_3$, R$_4$ identiques ou différents représentent un atome d'hydrogène ou un radical méthyle.

4 °) Complexes organomagnésiens solides selon la revendication 1, caractérisé en ce que :
n est égal à 0, 1, 2 ou 3,

5 °) Complexes organomagnésiens solides selon la revendication 1, caractérisé en ce que :
R$_5$ représente un groupe alkyle contenant 1 à 4 atomes de carbone.

6 °) Complexes organomagnésiens solides selon la revendication 1, caractérisé en ce qu'ils répondent à la formule :
(RMgX) $_m$ / N (-CH$_2$-CH$_2$-O-CH$_2$-CH$_2$-O-CH$_3$)$_3$
M, R, X ayant les significations précitées.

7 °) Procédé de préparation des complexes organomagnésiens solides selon la revendication 1, caractérisé en ce qu'on met en contact un solvant anhydre et sous atmosphère inerte un organomagnésien de formule RMgX avec un agent complexant de formule :
N [-CHR$_1$-CHR$_2$-O-(CHR$_3$-CHR$_4$-O) $_n$ R$_5$]$_3$
R, X, R$_1$, R$_2$, R$_3$, R$_4$, R$_5$ et n ayant les significations précitées

8 °) Procédé de préparation des complexes organomagnésiens solides selon la revendication 7, caractérisé en ce que le solvant anhydre choisi est de préférence l'éther éthylique ou le tétrahydrofuranne.

9 °) Procédé de préparation des comp    ganomagnésiens solides selon la revendication 7, caractérisé en ce qu'on utilise un rapport molaire du composé organomagnésien à l'agent complexant supérieur à 1.

10 °) Procédé de préparation des complexes organomagnésiens solides selon la revendication 7, caractérisé en ce que l'agent complexant est utilisé en solution dans l'éther éthylique ou le tétrahydrofuran-ne.

11 °) Application des complexes de formule générale (I), caractérisé en ce que l'on fait réagir un com-plexe de formule I selon la révendication 1 avec composé carbonylé en milieu solvant organique.

12 °) Application selon la revendication 11, caractérisée en ce que le composé carbonylé est choisi parmi les cétones, les aldéhydes, les esters, les chlorures et anhydrides d'acide.

13 °) Application selon la revendication 11, caractérisé en ce que le milieu solvant organique utilisé est choisi dans le groupe de solvants formé par l'éther éthylique, le tétrahydrofuranne, l'éther de pétrole, le cyclohexane, le toluène, l'acétonitrile, les alcanes, le diméthylacétamide, l'hexaméthylphosphoramide, le diméthylformamide.

**Claims**

1. Solid organomagnesium complexes, characterized in that they correspond to the general formula:
(RMgX)$_m$/ N [-CHR$_1$-CHR$_2$-O-(CHR$_3$-CHR$_4$-O)$_n$R$_5$]$_3$ (I)
in which:
m is equal to or greater than 1 and equal to or smaller than 3,
R denotes a group which is:
− linear or branched alkyl containing from 1 to 18 carbon atoms,

– alkenyl or alkynyl containing from 2 to 18 carbon atoms,

– cycloalkyl or cycloalkenyl containing from 3 to 12 carbon atoms,

– alkoxyalkyl, dialkoxyalkyl or alkylthiocarbonyl,

– aryl, position-substituted if desired at least by a halogen, or an alkoxy, trifluoromethyl, trifluoromethoxy or trifluoromethylthio group, or

– alkoxyaryl or alkylthioaryl,

X denotes a chlorine, iodine or bromine atom, and

n is an integer greater than or equal to 0 and smaller than or equal to 10 approximately $0 \leq n \leq 10$), $R_1$, $R_2$, $R_3$ and $R_4$, which are identical or different, denote a hydrogen atom or an alkyl or cycloalkyl radical containing from 1 to 4 carbon atoms, and $R_5$ denotes an alkyl or cycloalkyl radical containing 1 to 12 carbon atoms, a phenyl radical or a radical of formula $-C_pH_{2p}-\varnothing$ or $C_pH_{2p+1}-\varnothing-$, p being between 1 and approximately 12.

2. Solid organomagnesium complexes according to Claim 1, characterized in that:

R denotes an alkyl or alkenyl group containing at least 3 carbon atoms and substituted if desired by an alkoxy, dialkoxy, alkylthio or phenyl group.

3. Solid organomagnesium complexes according to Claim 1, characterized in that:

$R_1$, $R_2$, $R_3$ and $R_4$, which are identical or different, denote a hydrogen atom or a methyl radical.

4. Solid organomagnesium complexes according to Claim 1, characterized in that:

n is equal to 0, 1, 2 or 3.

5. Solid organomagnesium complexes according to Claim 1, characterized in that:

$R_5$ denotes an alkyl group containing 1 to 4 carbon atoms.

6. Solid organomagnesium complexes according to Claim 1, characterized in that they correspond to the formula:

$(RMgX)_m / N (-CH_2-CH_2-O-CH_2-O-CH_3)_3$

m, R and X having the abovementioned meanings.

7. Process for the preparation of the solid organomagnesium complexes according to Claim 1, characterized in that an organomagnesium compound of formula RMgX is brought into contact with a complexing agent of formula:

$N [-CHR_1-CHR_2-O-(CHR_3-CHR_4-O)_n R_5]_3$

R, X, $R_1$, $R_2$, $R_3$, $R_4$, $R_5$ and n having the abovementioned meanings, in an anhydrous solvent and under an inert atmosphere.

8. Process for the preparation of the solid organomagnesium complexes according to Claim 7, characterized in that the anhydrous solvent chosen is preferably ethyl ether or tetrahydrofuran.

9. Process for the preparation of the solid organomagnesium complexes according to Claim 7, characterized in that a molar ratio of the organomagnesium compound to the complexing agent which is greater than 1 is employed.

10. Process for the preparation of the solid organomagnesium complexes according to Claim 7, characterized in that the complexing agent is employed in solution in ethyl ether or tetrahydrofuran.

11. Application of the complexes of general formula (I), characterized in that a complex of formula I according to Claim 1 is reacted with a carbonyl compound in organic solvent medium.

12. Application according to Claim 11, characterized in that the carbonyl compound is chosen from ketones, aldehydes, esters and acid chlorides and anhydrides.

13. Application according to Claim 11, characterized in that the organic solvent medium employed is chosen from the group of solvents consisting of ethyl ether, tetrahydrofuran, petroleum ether, cyclohexane, toluene, acetonitrile, the alkanes, dimethylacetamide, hexamethylphosphoramide and dimethylformamide.

## Patentansprüche

1. Feste Organomagnesium-Komplexe, dadurch gekennzeichnet, daß sie der allgemeinen Formel:

$(RMgX)_m / N [-CHR_1-CHR_2-O-(CHR_3-CHR_4-O)_nR_5]_3$ (I)

entsprechen, in der:

m gleich oder größer als 1 und gleich oder kleiner als 3 ist,

R darstellt einen Rest:

– Alkyl, linear oder verzweigt mit 1 bis 18 Kohlenstoffatomen,

– Alkenyl oder Alkynyl mit 2 bis 18 Kohlenstoffatomen,

– Cycloalkyl oder Cycloalkenyl mit 3 bis 12 Kohlenstoffatomen,

– Alkoxyalkyl, Dialkoxyalkyl, Alkylthiocarbonyl,

– Aryl, gegebenenfalls substituiert in wenigstens einer Stellung durch ein Halogen, einen Alkoxy-, Trifluormethyl-, Trifluormethoxy-, Trifluormethylthiorest,

– Alkoxyaryl, Alkylthioaryl,

X ein Chlor-, Jod- oder Bromatom darstellt,

n eine ganze Zahl größer oder gleich 0 und kleiner oder gleich ungefähr 10 darstellt ($0 \leq n \leq 10$),

$R_1$, $R_2$, $R_3$ und $R_4$ identisch oder verschieden sind und jeweils ein Wasserstoffatom oder einen Alkyl-oder Cycloalkylrest mit 1 bis 4 Kohlenstoffatomen darstellen und $R_5$ einen Alkylrest oder Cycloalkylrest mit 1 bis 12 Kohlenstoffatomen, einen Phenylrest oder einen Rest der Formel $-C_pH_{2p}-\varnothing$ oder der Formel $C_pH_{2p+1}-\varnothing$ darstellt, wobei p zwischen 1 und ungefähr 12 liegt.

2. Feste Organomagnesium-Komplexe nach Anspruch 1, dadurch gekennzeichnet, daß:
R einen Alkylrest oder Alkenylrest mit wenigstens 3 Kohlenstoffatomen darstellt, gegebenenfalls substituiert mit einem Alkoxy-, Dialkoxy-, Alkylthio- oder Phenylrest.

3. Feste Organomagnesium-Komplexe nach Anspruch 1, dadurch gekennzeichnet, daß $R_1$, $R_2$, $R_3$, $R_4$ identisch oder verschieden sind und jeweils ein Wasserstoffatom oder einen Methylrest darstellen.

4. Feste Organomagnesium-Komplexe nach Anspruch 1, dadurch gekennzeichnet, daß n gleich 0, 1, 2 oder 3 ist.

5. Feste Organomagnesium-Komplexe nach Anspruch 1, dadurch gekennzeichnet, daß $R_5$ einen Alkylrest mit 1 bis 4 Kohlenstoffatomen darstellt.

6. Feste Organomagnesium-Komplexe nach Anspruch 1, dadurch gekennzeichnet, daß sie der Formel:
$(RMgX)_m$ / N $(-CH_2-CH_2-O-CH_2-CH_2-O-CH_3)_3$
entsprechen, in der m, R und X die vorgenannte Bedeutung haben.

7. Verfahren zur Herstellung von festen Organomagnesium-Komplexen nach Anspruch 1, dadurch gekennzeichnet, daß man ein wasserfreies Lösungsmittel und unter Inertatmosphäre eine Organomagnesium-Verbindung der Formel RMgX mit einem Komplexierungsagens der Formel:
N $[-CHR_1-CHR_2-O-(CHR_3-CHR_4-O)_nR_5]_3$
in Kontakt bringt, in der R, X, $R_1$, $R_2$, $R_3$, $R_4$, $R_5$ und n die vorgenannte Bedeutung haben.

8. Verfahren zur Herstellung von festen Organomagnesiumkomplexen nach Anspruch 7, dadurch gekennzeichnet, daß das gewählte wasserfreie Lösungsmittel vorzugsweise Ethylether oder Tetrahydrofuran ist.

9. Verfahren zur Herstellung von festen Organomagnesiumkomplexen nach Anspruch 7, dadurch gekennzeichnet, daß man ein molares Verhältnis von Organomagnesiumverbindung zu Komplexierungsagens von größer als 1 wählt.

10. Verfahren zur Herstellung von festen Organomagnesiumkomplexen nach Anspruch 7, dadurch gekennzeichnet, daß das Komplexierungsagens in Lösung in Ethylether oder Tetrahydrofuran eingesetzt wird.

11. Verwendung von Komplexen der allgemeinen Formel (I), dadurch gekennzeichnet, daß man einen Komplex der Formel (I) gemäß Anspruch 1 mit einer Carbonylverbindung in einem organischen Lösungsmittel reagieren läßt.

12. Verwendung nach Anspruch 11, dadurch gekennzeichnet, daß die Carbonylverbindung ausgewählt wird aus den Ketonen, den Aldehyden, den Estern, den Säurechloriden und -anhydriden.

13. Verwendung nach Anspruch 11, dadurch gekennzeichnet, daß das verwendete organische Lösungsmittel ausgewählt wird aus der Gruppe der Lösungsmittel gebildet aus Ethylether, Tetrahydrofuran, Petrolether, Cyclohexan, Toluol, Acetonitril, den Alkanen, Dimethylacetamid, Hexamethylphosphoramid und Dimethylformamid.